# EUROPEAN PATENT APPLICATION

(11) **EP 3 459 553 A1**
(43) Date of publication of application: **27.03.2019**
(21) Application number: 17799400.1
(22) Date of filing: 16.05.2017
(51) Int. Cl.: A61K 35/545, A61K 35/30, A61P 1/00, A61P 11/00, A61P 21/00, A61P 25/00, A61P 25/02, A61P 25/08, A61P 25/14, A61P 25/18, A61P 25/28, C12N 5/0775

(54) **AMELIORATION AND TREATMENT OF PERINATAL BRAIN DAMAGE WITH PLURIPOTENT STEM CELLS**

(30) Priority: 16.05.2016 JP 2016098186
(71) Applicant: National University Corporation Nagoya University, Nagoya-shi, Aichi 464-8601 (JP); Life Science Institute, Inc., Tokyo 100-8251 (JP)
(72) Inventor: SATO, Yoshiaki, Nagoya-shi Aichi 464-8601 (JP); SUZUKI, Toshihiko, Nagoya-shi Aichi 464-8601 (JP); SHIMIZU, Shinobu, Nagoya-shi Aichi 464-8601 (JP); MIZUNO, Masaaki, Nagoya-shi Aichi 464-8601 (JP); HAYAKAWA, Masahiro, Nagoya-shi Aichi 464-8601 (JP); DEZAWA, Mari, Sendai-shi Miyagi 980-8575 (JP)
(74) Representative: AWA Sweden AB
(86) International application number: PCT/JP2017/018416
(87) International publication number: WO 2017/199976

(57) **Abstract**

The present invention addresses the problem of providing a novel use of pluripotent stem cells (Muse cells) for medical purposes in regenerative medicine. The present invention provides a cell preparation and a pharmaceutical composition both for ameliorating and treating perinatal brain damage including learning disability and motor disability, each of the cell preparation and the pharmaceutical composition containing SSEA-3-positive pluripotent stem cells isolated from a mesenchymal tissue collected from a living body or cultured mesenchymal cells. The cell preparation according to the present invention relies on a mechanism that Muse cells are administered to a subject having the above-mentioned damage to cause the engraftment of the Muse cells in a damaged brain tissue, thereby ameliorating and treating the damage.

## Description

### FIELD

The present invention relates to a cell preparation for regenerative medicine. More specifically, it relates to an effective cell preparation for treatment of perinatal brain damage containing pluripotent stem cells, and to a novel treatment method.

### BACKGROUND

Perinatal brain damage refers to brain damage such as cerebral palsy, mental development delay, epilepsy or sensory disorder that is caused by some abnormality arising during the fetal or neonatal period, and its causes and pathology are still incompletely understood. Research has recently been progressing in the field of perinatal medicine in regard to fetal hypoxia, leading to greater focus on hypoxia as a cause of damage. When hypoxia progresses, the fetus exhibits various metabolic reactions and further falls into a state of metabolic insufficiency, resulting in damage to the organs including the brain, with death expected to occur in some cases. Preventing hypoxia during delivery is therefore thought to be clearly linked with preventing perinatal brain damage including cerebral palsy. However, it is difficult to prevent hypoxia during delivery, and it is said that hypoxia is responsible for approximately 20% of all cerebral palsy cases (NPL 1).

Research has recently been progressing in the field of regenerative medicine on a host of cell therapies using stem cells, and as clinical applications begin to emerge, it is hoped that stem cells will be used for improvement and treatment of perinatal brain damage (NPL 2). Embryonic stem cells (ES cells), neural stem/progenitor cells (NSPC), induced pluripotent stem cells (iPS cells) and umbilical cord blood stem cells (UCBC) are known types of stem cells that are expected to have potential in clinical applications for central nervous system diseases related to perinatal brain damage. The present inventors have previously co-administered proliferated NSPCs cultured from rat fetal brains together with a chondroitin sulfate-degrading enzyme into the cerebral ventricle of a perinatal hypoxic ischemic encephalopathy (HIE) rat model, and found that the infarct area decreases significantly compared to an untreated group and to a group administered neural stem cells alone (NPL 3). It has also been confirmed by the present inventors that intraperitoneal administration of UCBCs to an HIE rat model temporarily reduces hypoxic ischemic encephalopathy.

The bone marrow-derived mesenchymal cells (MSCs) fraction has been isolated from adults, and it is known to have the ability to differentiate into bone, cartilage, adipocytes, neurons and skeletal muscle, for example (NPL 4 and 5). However, MSCs are heterogeneous cell populations, the nature of their differentiation potency is unknown, and they have wide variation in therapeutic effect. iPS cells (PTL 1) have been reported as adult pluripotent stem cells, but establishing iPS cells requires the very complex procedure of introducing specific genes or specific compounds into the skin fibroblast fraction (mesenchymal cell fraction) of somatic cells, while iPS cells could also have high tumor-forming potential, and therefore high hurdles stand in the way of their clinical application.

Research by Prof. Dezawa, one of the present inventors, has demonstrated that the pluripotency of the mesenchymal cells fraction is exhibited by pluripotent stem cells (Multilineage-differentiating Stress Enduring cells, or Muse cells) that express SSEA-3 (Stage-Specific Embryonic Antigen-3) as a surface antigen, which are present in the mesenchymal cell fraction and can be obtained without operation of induction and that this holds potential for application in treatment of diseases by tissue regeneration. It has also been found that Muse cells can be enriched by treating the mesenchymal cells fraction with one or more of different types of stress treatments (PTL 2, NPL 6). However, it has not yet been demonstrated that the expected therapeutic effect can be obtained using Muse cells for amelioration and/or treatment of perinatal brain damage.

### [CITATION LIST]

### [PATENT LITERATURE]

[PTL 1] Japanese Patent Publication No. 4183742
[PTL 2] International Patent Publication No. WO2011/007900

### [NON PATENT LITERATURE]

[NPL 1] Ikeda, T., Nou to Hattatsu, Vol.43, p.206-210 or 2011
[NPL 2] Sato, Y., Shusanki Igaku, Vol.41, 1531-1536, 2011
[NPL 3] Sato, Y., et al., Report Sci., Vol.15, p.613-620(2008)
[NPL 4] Dezawa, M., et al., J. Clin. Invest., Vol.113, p.1701-1710(2004)
[NPL 5] Dezawa, M., et al., Science, Vol.309, p.314-317(2005)
[NPL 6] Wakao, S, et al., Proc. Natl. Acad. Sci. USA, Vol.108, p.9875-9880(2011)

### SUMMARY

### [TECHNICAL PROBLEM]

It is an object of the present invention to provide a novel medical use for pluripotent stem cells (i.e., Muse cells) in regenerative medicine. More specifically, it is an object of the present invention to provide a cell preparation and pharmaceutical composition that include Muse cells and are effective for treating perinatal brain damage, as well as a novel treatment method.

### [SOLUTION TO PROBLEM]

The present inventors have found that preparing a perinatal hypoxic-ischemic encephalopathy (HIE) rat model, and administering Muse cells by intravenous injection 72 hours after hypoxic treatment, ameliorates perinatal brain damage (including learning disability and motor disability, for example), and the present invention has thus been completed.

Specifically, the present invention provides the following.
[1] A cell preparation for amelioration and/or treatment of perinatal brain damage, comprising pluripotent stem cells positive for SSEA-3 isolated from mesenchymal tissue of a body or cultured mesenchymal cells.
[2] The cell preparation according to [1] above, comprising a cell fraction wherein pluripotent stem cells positive for SSEA-3 have been concentrated by external stress treatment.
[3] The cell preparation according to [1] or [2] above, wherein the pluripotent stem cells are CD105-positive.
[4] The cell preparation according to any one of [1] to [3] above, wherein the pluripotent stem cells are CD117-negative and CD146-negative.
[5] The cell preparation according to any one of [1] to [4] above, wherein the pluripotent stem cells are CD117-negative, CD146-negative, NG2-negative, CD34-negative, vWF-negative and CD271-negative.
[6] The cell preparation according to any one of [1] to [5] above, wherein the pluripotent stem cells are CD34-negative, CD117-negative, CD146-negative, CD271-negative, NG2-negative, vWF-negative, Sox10-negative, Snail-negative, Slug-negative, Tyrp1-negative and Dct-negative.
[7] The cell preparation according to any one of [1] to [6] above, wherein the pluripotent stem cells have all of the following properties:
   (i) low or non-existent telomerase activity;
   (ii) having the ability to differentiate into any of the three germ layers;
   (iii) exhibiting no neoplastic proliferation; and
   (iv) having self-renewal ability.
[8] The cell preparation according to any one of [1] to [7] above, wherein the perinatal brain damage is selected from the group consisting of learning disability, motor disability, cerebral palsy, behavior disorder, mental development abnormality, sensory disorder, speech disorder, epilepsy, dysphagia and abnormal respiratory control.
[9] The cell preparation according to any one of [1] to [8] above, wherein the pluripotent stem cells have the ability to engraft into brain tissue.
[10] The cell preparation according to any one of [1] to [9] above, which is to be administered to a human neonate, infant or child with the pluripotent stem cells at from approximately 1 × 10⁵ cells/individual to approximately 1 × 10⁸ cells/individual, as the therapeutically effective amount.
[11] The cell preparation according to any one of [1] to [10] above, which is to be administered to a human neonate, infant or child with the pluripotent stem cells in an amount of cells per body weight of approximately 3 × 10⁴ cells/kg to approximately 3 × 10⁷ cells/kg per target individual, as the therapeutically effective amount.

### [ADVANTAGEOUS EFFECTS OF INVENTION]

The present invention can drastically minimize perinatal brain damage in a subject (child) suffering from perinatal brain damage, by a brain tissue-regenerating mechanism in which Muse cells are administered through the veins or by another route to selectively accumulate them in damaged brain tissue, and the Muse cells differentiate to brain tissue-forming cells within the tissue.

### BRIEF DESCRIPTION OF DRAWINGS

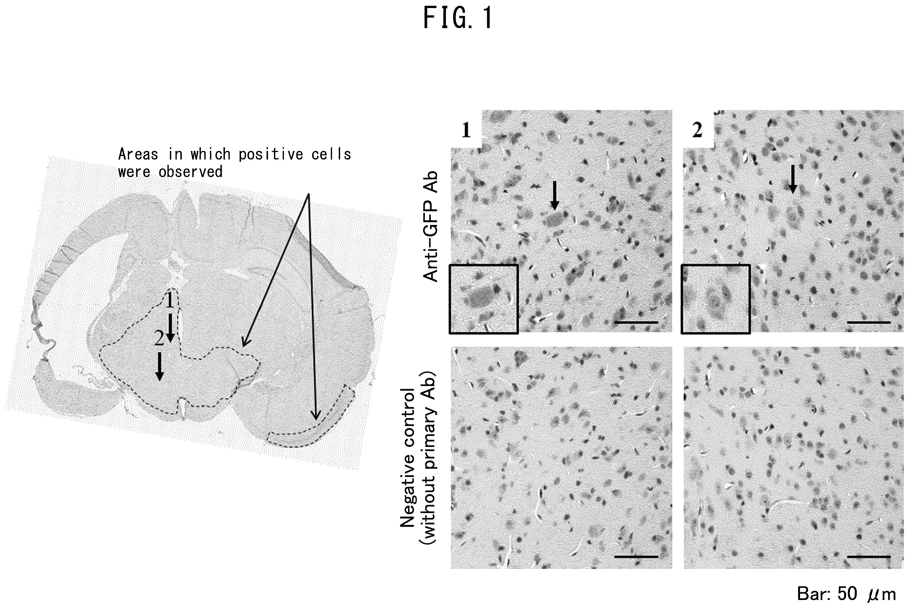

FIG. 1 shows the results of confirming engraftment of Muse cells in the brain tissue when administered to an HIE rat model (postnatal age of 48 days; 10 days after administration).

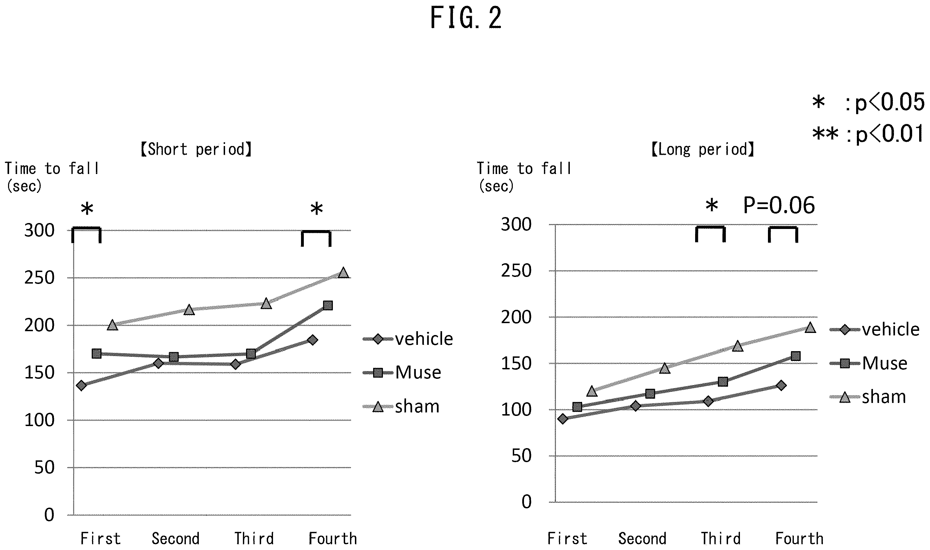

FIG. 2 shows the results of a Rota-rod treadmill test for motor function in an HIE rat model, using a Muse cell-administered group ("Muse"), a group with addition of a cell suspension (physiological saline) alone ("vehicle") and a sham-operated group ("sham") ("Short period": 1 month after birth; "Long period": 5 months after birth).

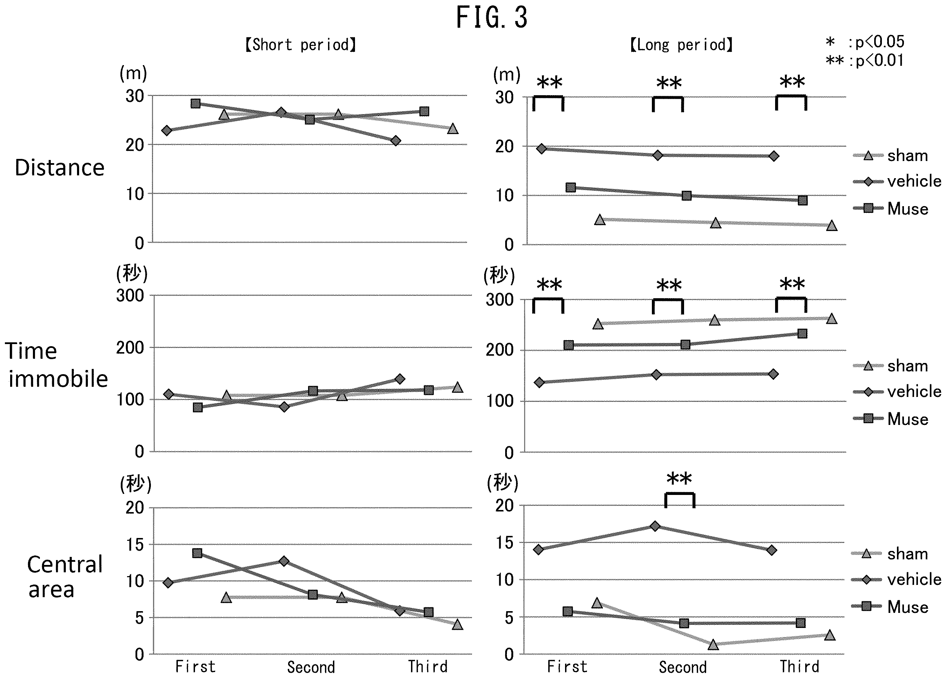

FIG. 3 shows the results of an open field test for emotional behavior (hyperactivity, etc.) in an HIE rat model, using a Muse cell-administered group ("Muse"), a group with addition of a cell suspension (physiological saline) alone ("vehicle") and a sham-operated group ("sham") ("Short period": 1 month after birth; "Long period": 5 months after birth).

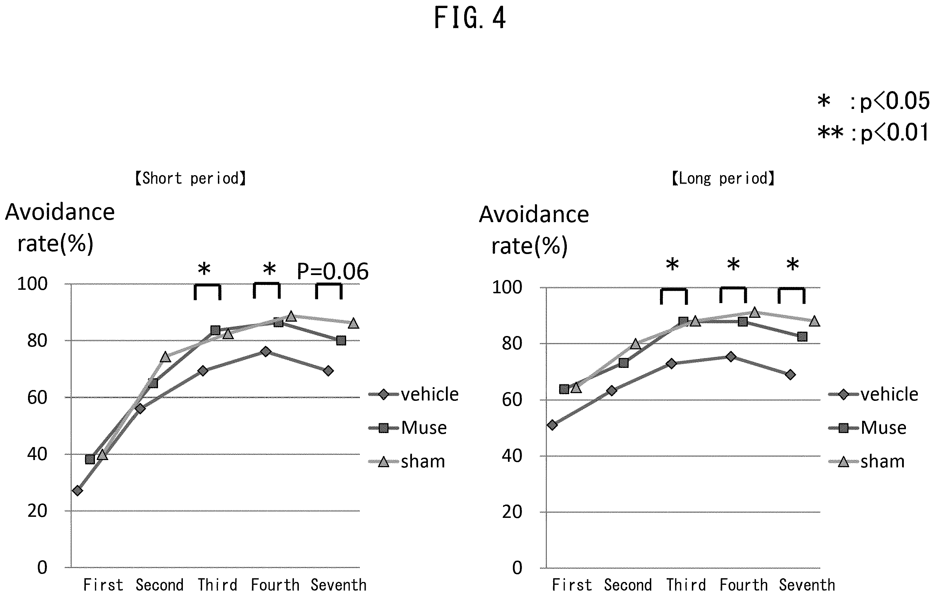

FIG. 4 shows the results of a shuttle avoidance test for improvement in learning disability in an HIE rat model, using a Muse cell-administered group ("Muse"), a group with addition of a cell suspension (physiological saline) alone ("vehicle") and a sham-operated group ("sham") ("Short period": 1 month after birth; "Long period": 5 months after birth).

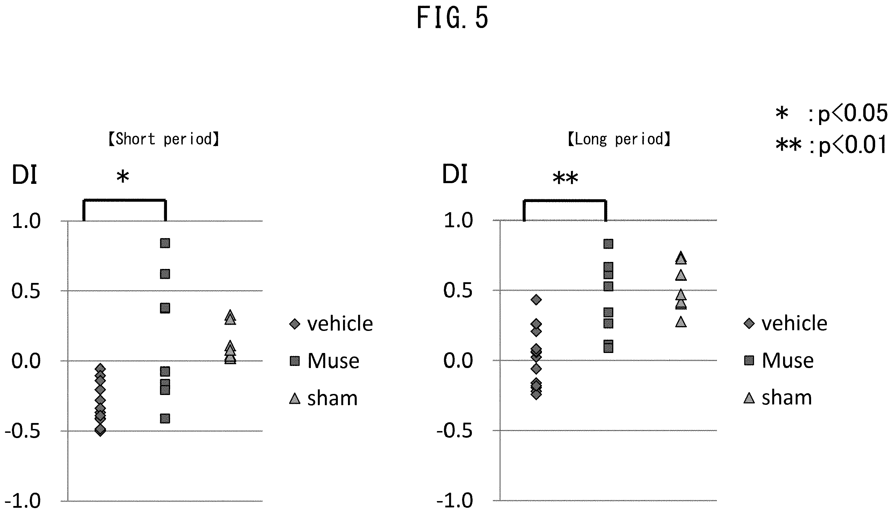

FIG. 5 shows the results of a novel object recognition test for improvement in memory learning and visual cognitive memory in an HIE rat model, using a Muse cell-administered group ("Muse"), a group with addition of a cell suspension (physiological saline) alone ("vehicle") and a sham-operated group ("sham") ("Short period": 1 month after birth; "Long period": 5 months after birth).

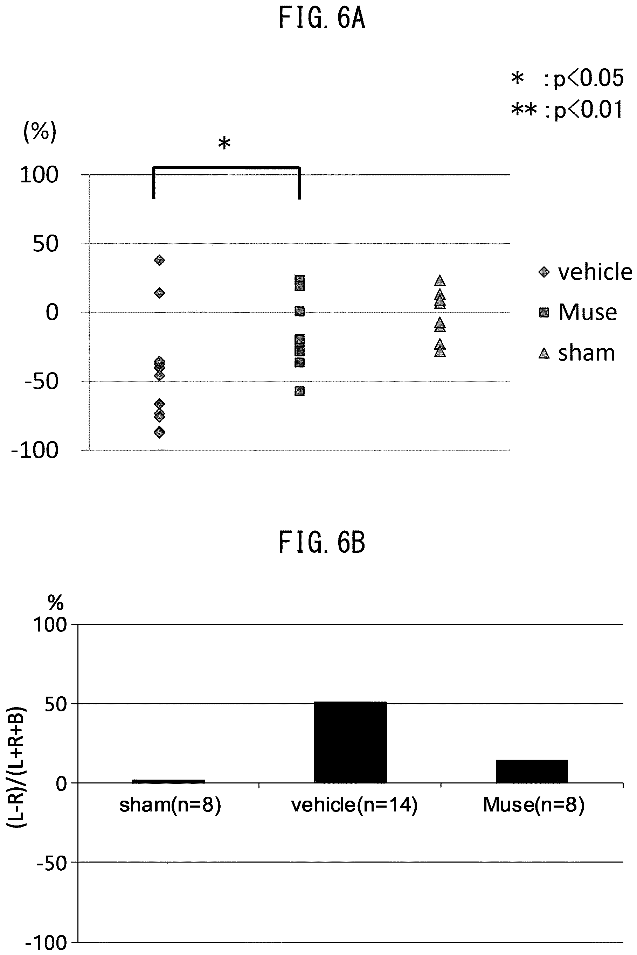

FIG. 6A shows the results of a cylinder test for improvement in motor function in an HIE rat model, using a Muse cell-administered group ("Muse"), a group with addition of a cell suspension (physiological saline) alone ("vehicle") and a sham-operated group ("sham") (5 months after birth).
FIG. 6B shows the results of a cylinder test for improvement in motor function in an HIE rat model, using a Muse cell-administered group ("Muse"), a group with addition of a cell suspension (physiological saline) alone ("vehicle") and a sham-operated group ("sham") (5 months after birth).

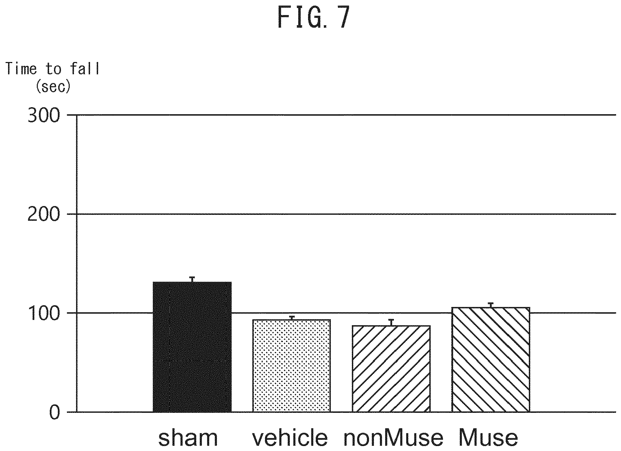

FIG. 7 shows the results of a Rota-rod treadmill test for motor function in an HIE rat model, using a Muse cell-administered group ("Muse"), a non-Muse cell-administered group ("non Muse"), a group with addition of a cell suspension (physiological saline) alone ("vehicle") and a sham-operated group ("sham") (5 months after birth).

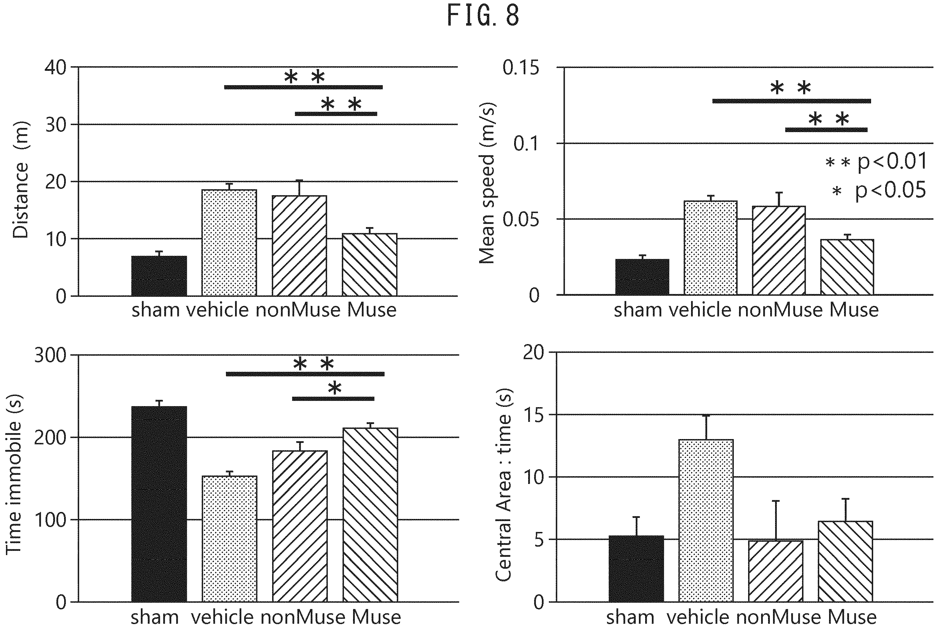

FIG. 8 shows the results of an open field test for emotional behavior (hyperactivity, etc.) in an HIE rat model, using a Muse cell-administered group ("Muse"), a non-Muse cell-administered group ("non Muse"), a group with addition of a cell suspension (physiological saline) alone ("vehicle") and a sham-operated group ("sham") (5 months after birth).

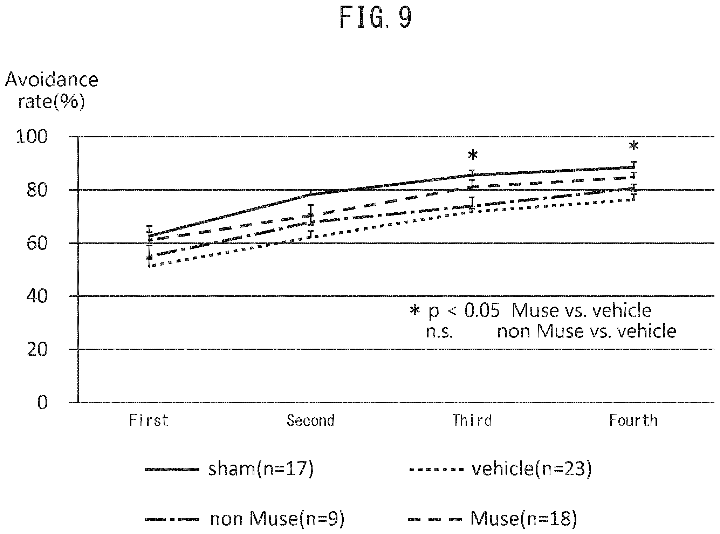

FIG. 9 shows the results of a shuttle avoidance test for improvement in learning disability in an HIE rat model, using a Muse cell-administered group ("Muse"), a non-Muse cell-administered group ("non Muse"), a group with addition of a cell suspension (physiological saline) alone ("vehicle") and a sham-operated group ("sham") (5 months after birth).

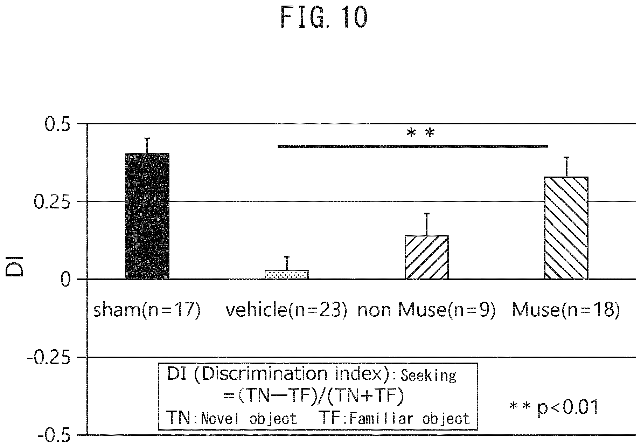

FIG. 10 shows the results of a novel object recognition test for improvement in memory learning and visual cognitive memory in an HIE rat model, using a Muse cell-administered group ("Muse"), a non-Muse cell-administered group ("non Muse"), a group with addition of a cell suspension (physiological saline) alone ("vehicle") and a sham-operated group ("sham") (5 months after birth).

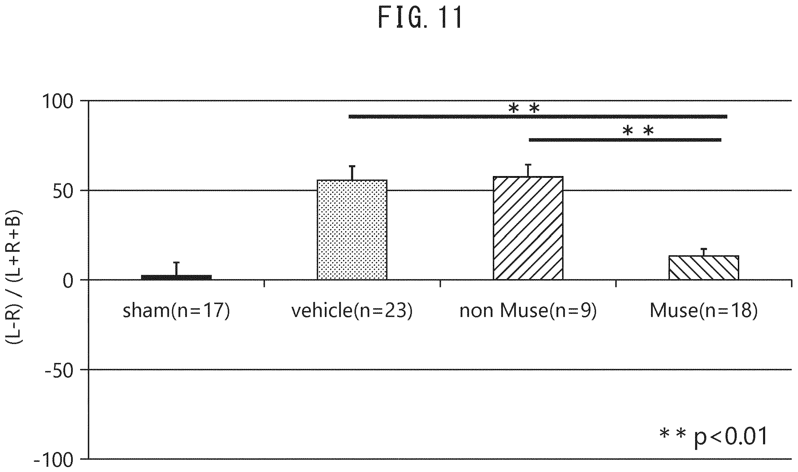

FIG. 11 shows the results of a cylinder test for improvement in motor function in an HIE rat model, using a Muse cell-administered group ("Muse"), a non-Muse cell-administered group ("non Muse"), a group with addition of a cell suspension (physiological saline) alone ("vehicle") and a sham-operated group ("sham") (5 months after birth).

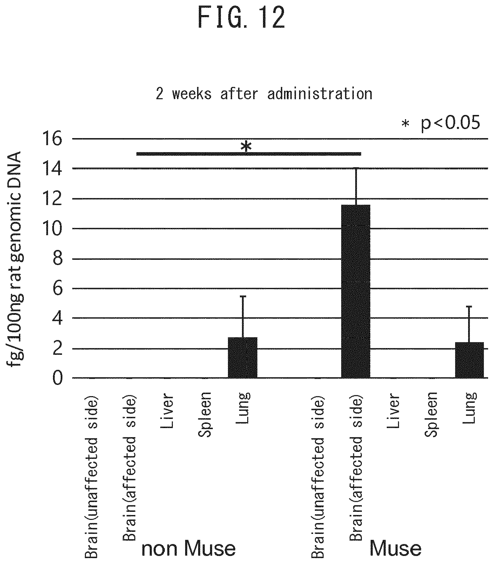

FIG. 12 shows the results for engraftment volume in each tissue 2 weeks after administration to an HIE rat model, using a Muse cell-administered group ("Muse") and a non-Muse cell-administered group ("non Muse").

### DESCRIPTION OF EMBODIMENTS

The present invention relates to a cell preparation and pharmaceutical composition for amelioration and/or treatment of perinatal brain damage, containing SSEA-3 positive pluripotent stem cells (Muse cells), and to a novel treatment method. The present invention will now be explained in greater detail.

### 1. Applicable diseases and their diagnosis

The present invention is directed toward amelioration and treatment of perinatal brain damage using a cell preparation or pharmaceutical composition containing SSEA-3 positive pluripotent stem cells (Muse cells). The term "perinatal brain damage" generally refers to brain damage occurring in the perinatal period (in humans, the period from 22 weeks after pregnancy until up to 7 days after child birth), and it means, for example, brain damage associated with intrapartum hypoxic-ischemic encephalopathy or successive systemic inflammatory response syndrome due to viral or bacterial infection. According to the present invention, however, the period during which cerebral palsy caused by hypoxic-ischemic encephalopathy manifests as a symptom (for example, 2-3 years of age for humans) is also within the range of application. More specifically, according to the present invention, the target of amelioration and treatment is perinatal brain damage produced in human neonates (up to 28 days after birth), infants (up to 1 year after birth) and children (1 to 6 years after birth).

The term "hypoxic-ischemic encephalopathy" refers to brain damage due to low oxygen and reduced flow of arterial blood, which is a cause of neonatal death, cerebral palsy and mental retardation. The causes include hypoxia resulting from anesthesia, cardiac failure or carbon monoxide poisoning of the mother during the fetal stage, hypotension due to lumbar anesthesia or inferior aortic compression by the uterus, hypertonic uterine dysfunction due to overdosage of oxytocin (a labor inducing agent), umbilical cord blood flow disturbance, placental dysfunction and placental abruption. It may also be caused by hypoxia due to severe postpartum hemorrhage, shock, brain damage, anesthesia, trauma, congenital heart disease or pulmonary insufficiency. In mature infants (in humans, this refers to neonates having a birth weight of about 3,000 g and body height of about 50 cm, having developed after elapse of 10 months in the womb to a state in which they can survive outside of the womb), it leads to cerebral cortical necrosis or parasagittal ischemia, and in premature infants it leads to periventricular softening or intraventricular hemorrhage. Cerebral edema is a complication in severe cases. This condition is characterized by pallid skin, cyanosis, apnea, bradycardia, reduced muscle tone and unresponsiveness to stimuli. Cerebral edema may occur within 24 hours after birth, resulting in brain stem compression. Prognosis is poor, and death, cerebral palsy, mental retardation and severe physical or mental disorder may result.

The term "cerebral palsy" refers to irreversible brain damage produced during the developmental stage of the brain (in humans, this is the period from the 13th day of pregnancy to a postnatal age of 48 days), being associated with nonprogressive lesions, and one of its signs being motor function disorder which usually manifests by 3 years of age. More specifically, it generally refers to brain damage occurring up to the neonatal period. The causes are divided according to the period in which damage occurs, namely: (a) prenatal causes such as intrauterine infection, placental dysfunction, fetal cerebrovascular disease and hereditary causes, (b) causes at birth such as intrapartum mechanical injury, cerebral hemorrhage, anoxia, hypoxia and cerebral circulation disturbance, and (c) postnatal causes such as severe jaundice (nuclear icterus), intracranial infection and cerebral hemorrhage. Classification depends on the type of paralysis. Muscle tone problems include ankylosis (spasm), stiffness, disorder, athetosis (maintaining certain postures, or involuntary movements taking place with intention of movement) and atonia, and broadened paralysis may include quadriplegia, hemiplegia, diplegia, paraplegia, double hemiplegia and monoplegia. Complications include intellectual disability (including learning disability), epileptic seizure, cranial nerve damage and speech disorder. The brain lesions caused by hypoxic-ischemic encephalopathy in mature infants are usually cerebral cortical stratified necrosis, basal nuclear necrosis, cerebral infarction, white matter softening or bridging hilar necrosis, with necrosis of the brain stem also often observed, and clinically, basal nuclear necrosis is a cause of athetoid-type cerebral palsy while cerebral infarction is a cause of spasmodic quadriplegia and hemiplegia. Brain stem necrosis usually has poor prognosis and leads to death in infancy, and even with survival, it results in dysphagia and abnormal respiratory control.

Diagnosis of an applicable disease for amelioration and treatment with the cell preparation and pharmaceutical composition of the present invention is first carried out by a physician according to a hypothermia therapy entry criteria flow chart, since hypothermia therapy is effective for neonatal hypoxic-ischemic encephalopathy. More specifically, diagnosis is based on whether or not the neurological symptoms at child birth fall under the assessment items listed as criteria A (objective finding of whole body hypoxia or ischemia), and criteria B (subjective finding of encephalopathy). When criteria A and criteria B apply, a diagnosis of perinatal brain damage may be made.
Criteria A: Child birth after at least 36 weeks of gestation, and satisfying at least one of the following conditions.
   - Apgar score of ≤5 at 10 minutes after birth
   - Requirement for continuous neonatal resuscitation (tracheal intubation, positive pressure ventilation, etc.) for 10 minutes or longer
   - pH of <7.0 in blood gas (umbilical cord blood, artery, vein, peripheral capillary) within 60 minutes after birth
   - Base deficit of 16 mmol/L or greater in blood gas (umbilical cord blood, artery, vein, peripheral capillary) within 60 minutes after birth
   Neonates satisfying criteria A are then evaluated for the presence of abnormalities by neurological clinical examination for B.
Criteria B: Moderate to severe encephalopathy (corresponding to a Sarnat classification of ≥2), i.e. impaired consciousness (somnolence, torpor, coma) and at least one of the following symptoms (preferably by inspection by a neonatologist or pediatric neurologist highly familiar with neonatal hypoxic-ischemic encephalopathy)
   - Reduced muscle tone
   - Abnormal reflex including "doll's eye" reflection or pupil reflex abnormality
   - Reduced or absent sucking reflex
   - Clinical spasm

Ultrasonography, MRI, CT, electroencephalogram and laser Doppler blood flow meter analysis may also be used if necessary in order to obtain physiological findings pertaining to the brain. Perinatal brain damage may be diagnosed when an abnormal finding is obtained using such apparatuses. Diagnosis of an applicable disease can also be made based on direct observation of learning disability or motor disability. According to the present invention, the cell preparation and pharmaceutical composition described below are administered to (or "transplanted in", as it may also be termed hereunder) a subject for treatment of the applicable disease, to allow amelioration and/or treatment of the applicable disease. The term "amelioration" here means alleviating or suppressing progression of symptoms accompanying perinatal brain damage, and preferably it means alleviating symptoms to an extent that is not a problem for daily living activities. The term "treatment" refers to suppressing or completely eliminating symptoms of perinatal brain damage.

### 2. Cell preparation and pharmaceutical composition

### (1) Pluripotent stem cells

The pluripotent stem cells to be used in the cell preparation and pharmaceutical composition of the present invention are typically cells whose existence in the human body was discovered by Prof. Dezawa, one of the present inventors, and which are named "Muse (Multilineage-differentiating Stress Enduring) cells". Muse cells can be obtained from bone marrow fluid and adipose tissue (Ogura, F., et al., Stem Cells Dev., Nov 20, 2013 (Epub) (published on Jan 17, 2014)) or from skin tissue such as dermal connective tissue, and they are widely dispersed throughout the connective tissue of various organs. The cells have the properties of both pluripotent stem cells and mesenchymal stem cells, and are identified as being double-positive for the cell surface markers "SSEA-3 (Stage-specific embryonic antigen-3)" and "CD105". Therefore, Muse cells or cell populations containing Muse cells, for example, can be isolated from body tissue using these antigen markers. Muse cells are also stress-tolerant, and can be concentrated from mesenchymal tissue or cultured mesenchymal cells by different types of stress treatments. A cell fraction with Muse cells enriched by stress treatment may be used as the cell preparation of the present invention. The methods of separation and identification of Muse cells, and their features, are disclosed in detail in International Patent Publication No. WO2011/007900. Also, as reported by Wakao et al. (2011, ibid.), when mesenchymal cells are cultured from the bone marrow or skin and used as a parent population of Muse cells, all of the SSEA-3 positive cells are also CD105-positive. Consequently, when Muse cells are isolated from mesenchymal tissue of a body or cultured mesenchymal stem cells for the cell preparation and pharmaceutical composition of the present invention, the Muse cells may be used after purification with SSEA-3 alone as the antigen marker. Throughout the present specification, pluripotent stem cells (Muse cells) or a cell population containing Muse cells, isolated from mesenchymal tissue of a body or cultured mesenchymal tissue using SSEA-3 as the antigen marker, and which can be used in a cell preparation and pharmaceutical composition for amelioration and/or treatment of perinatal brain damage, may be referred to simply as "SSEA-3 positive cells". Also throughout the present specification, "non-Muse cells" refers to cells that are present in mesenchymal tissue of a body or cultured mesenchymal tissue, and are the remainder of "SSEA-3 positive cells". In the Examples provided below, the non-Muse cells used are cell populations obtained by removing the SSEA-3 and CD105-positive cells from MSC by the method described in International Patent Publication No. WO2011/007900 for separation and identification of human Muse cells.

In brief, Muse cells or a cell population containing Muse cells can be isolated from body tissue (for example, mesenchymal tissue) using only antibody for the cell surface marker SSEA-3, or using antibodies for both SSEA-3 and CD105. The term "body" here means "mammalian body". According to the present invention, the "body" does not include a fertilized ovum or an embryo at a developmental stage before the blastocyst stage, but it does include an embryo at the developmental stage from the blastocyst stage onward, including the fetus or blastocyst. The mammal is not limited and may be a primate such as human or monkey, a rodent such as a mouse, rat, rabbit or guinea pig, or a cat, dog, sheep, pig, cow, horse, donkey, goat or ferret. The Muse cells to be used in the cell preparation and pharmaceutical composition of the present invention are clearly distinguished from embryonic stem cells (ES cells) or iPS cells based on separation from body tissue using a direct marker. The term "mesenchymal tissue" refers to tissue from the bone, synovial membrane, fat, blood, bone marrow, skeletal muscle, dermis, ligament, tendon, dental pulp, umbilical cord or umbilical cord blood, or tissues present in various organs. For example, the Muse cells may be obtained from the bone marrow or skin or adipose tissue. Preferably, mesenchymal tissue of a body is harvested and the Muse cells are isolated from the tissue and used. The separating means mentioned above may be used to separate Muse cells from cultured mesenchymal cells such as fibroblasts or bone marrow-derived MSCs. The Muse cells to be used for the cell preparation and pharmaceutical composition of the present invention may be either autologous or allogenic with respect to the recipient.

As mentioned above, Muse cells or a cell population containing Muse cells can be isolated from body tissue using SSEA-3 positivity, or double positivity for SSEA-3 and CD105, as indicators, but human adult skin is known to include various types of stem cells and progenitor cells. However, Muse cells are not identical to these cells. Such stem cells and progenitor cells include skin-derived precursors (SKP), neural crest stem cells (NCSC), melanoblasts (MB), perivascular cells (PC), endothelial precursor cells (EP) and adipose-derived stem cells (ADSC). Muse cells can be separated out as being "non-expressing" for the markers unique to these cells. More specifically, Muse cells can be separated by using non-expression for at least one, and for example, 2, 3, 4, 5, 6, 7, 8, 9, 10 or 11 among 11 markers selected from the group consisting of CD34 (EP and ADSC marker), CD117 (c-kit) (MB marker), CD146 (PC and ADSC marker), CD271 (NGFR) (NCSC marker), NG2 (PC marker), vWF factor (von Willebrand factor) (EP marker), Sox10 (NCSC marker), Snail (SKP marker), Slug (SKP marker), Tyrp1 (MB marker) and Dct (MB marker). As a non-limitative example, non-expression of CD117 and CD146 may be used as the indicator for separation, non-expression of CD117, CD146, NG2, CD34, vWF and CD271 may be used as the indicator, or non-expression of all of the aforementioned 11 markers may be used as the indicator for separation.

The Muse cells having the aforementioned features to be used for the cell preparation and pharmaceutical composition of the present invention may have at least one property selected from the group consisting of the following:
(i) low or non-existent telomerase activity;
(ii) having the ability to differentiate into any of the three germ layers;
(iii) exhibiting no neoplastic proliferation; and
(iv) having self-renewal ability.

According to one aspect of the present invention, the Muse cells to be used for the cell preparation and pharmaceutical composition of the present invention have all of these properties. As regards (i), "low or non-existent telomerase activity", this refers to low or non-detectable telomerase activity when using a TRAPEZE XL telomerase detection kit (Millipore), for example. "Low" telomerase activity is, for example, either telomerase activity on the same level as human fibroblasts, which are somatic cells, or telomerase activity of 1/5 and preferably no greater than 1/10 of that of Hela cells. In regard to (ii), the Muse cells have the ability to differentiate into the three germ layers (endoderm, mesoderm and ectoderm) *in vitro* and *in vivo,* and by induction culturing *in vitro,* for example, they can differentiate into hepatocytes, neurons, skeletal muscle cells, smooth muscle cells, osteocytes or adipocytes. They may also exhibit the ability to differentiate into the three germ layers in the case of transplanting *in vivo* into the testes. They also have the ability to migrate and engraft onto damaged organs (heart, skin, spine, liver, muscle, etc.), by administration into the body via intravenous injection, and differentiate into specific cells of the corresponding tissue. In regard to (iii), the Muse cells have the property of proliferating at a rate of about every 1.3 days in suspension culture, and growing in suspension culture from a single cell to form an embryoid-like cell mass, slow down the growth at about 14 days; however, when the embryoid-like cell mass is carried into adhesion culture, cell growth resumes and the proliferated cells spread out from the cell mass. They also have the property of not generating teratomas at least for 6 months after transplantation into the testes. In regard to (iv), Muse cells have self-renewal (auto-replicating) ability. The term "self-renewal" means that cells in the embryoid-like cell mass obtained by culturing a single Muse cell in suspension culture can be confirmed to differentiate into cells of all 3 germ layers, and also that when a single cell from the embryoid-like cell mass is again carried into a suspension culture, it forms a next generation embryoid-like cell mass, and reproduce differentiation into three germ layers as well as embryoid-like cell mass in the suspension culture can be confirmed. Self-renewal may be observed once or as several repeated cycles.

In addition, a cell fraction containing Muse cells to be used in the cell preparation of the present invention may be a cell fraction having the SSEA-3 positive and CD105-positive pluripotent stem cells concentrated, obtained by a method of applying external stress treatment to mesenchymal tissue of a body or cultured mesenchymal cells, causing the cells other than the external stress-resistant cells to die, and recovering the surviving cells, the cell fraction having at least one and preferably all of the following properties.
(i) SSEA-3 positivity;
(ii) CD105-positivity;
(iii) low or non-existent telomerase activity;
(iv) having the ability to differentiate into any of the three germ layers;
(v) exhibiting no neoplastic proliferation; and
(vi) having self-renewal ability.

The external stress may be any one or a combination of: protease treatment, culturing in a low oxygen concentration, culturing under low-phosphate conditions, culturing with low serum concentration, culturing under low nutritive conditions, culturing under exposure to heat shock, culturing at low temperature, freezing treatment, culturing in the presence of a hazardous substance, culturing in the presence of active oxygen, culturing under mechanical stimulation, culturing with agitating treatment, culturing with pressure treatment, or physical impact. For example, the treatment time with a protease is preferably a total of 0.5 to 36 hours to apply external stress to the cells. The protease concentration may be the concentration used when the cells adhering to a culture vessel are detached, when the cell mass is dispersed into individual cells, or when individual cells are recovered from tissue. The protease is preferably a serine protease, aspartic acid protease, cysteine protease, metalloprotease, glutamic acid protease or N-terminal threonine protease. The protease is also preferably trypsin, collagenase or dispase.

Muse cells having the aforementioned features, which are to be used in the cell preparation of the present invention, are administered to the body by intravenous administration or the like, after which they engraft onto damaged brain tissue, as described below. It is thought that the Muse cells differentiate into tissue-compatible cells thus ameliorating and/or treating perinatal brain damage.

### (2) Preparation and use of cell preparation and pharmaceutical composition

Although not limited thereto, the cell preparation and pharmaceutical composition of the present invention may be obtained by suspending the Muse cells or a cell population containing Muse cells obtained by (1) above, in physiological saline or an appropriate buffer (for example, phosphate-buffered physiological saline). In this case, when the number of Muse cells isolated from autologous or allogenic tissue is low, the cells may be cultured before administration for growth until the prescribed cell density is obtained. As already reported (International Patent Publication No. WO2011/007900), Muse cells do not undergo neoplastic transformation, and therefore even if the cells recovered from body tissue are in undifferentiated form, they have low tumorigenicity and are safe. There are no particular restrictions on culturing of the recovered Muse cells, and it may be carried out in ordinary growth medium (for example, α-Minimal Essential Medium (α-MEM) containing 10% newborn calf serum). More specifically, referring to International Patent Publication No. WO2011/007900, suitable medium and additives (for example, antibiotics and serum) may be selected for culturing and growth of the Muse cells, and a solution containing the prescribed density of Muse cells may be prepared. When a cell preparation or pharmaceutical composition of the present invention is to be administered to a human patient, roughly several milliliters of bone marrow fluid may be harvested from human iliac bone, and for example, the bone marrow-derived MSCs may be cultured as adherent cells from the bone marrow fluid to increase them to a number of cells allowing separation of an effective therapeutic amount of Muse cells, after which the Muse cells may be separated out with SSEA-3 antigen marker as the indicator, and autologous or allogenic Muse cells prepared as a cell preparation. As an alternative example, Muse cells that have been separated using SSEA-3 antigen marker as the indicator, and the cells cultured to increase them to an effective therapeutic amount, may then be prepared as a cell preparation of autologous or allogenic Muse cells.

For use of the Muse cells in a cell preparation or pharmaceutical composition, dimethyl sulfoxide (DMSO) or serum albumin may be added to the cell preparation or pharmaceutical composition to protect the cells, and an antibiotic or the like may be added to prevent infiltration and growth of bacteria. In addition, other pharmaceutically acceptable components (for example, carriers, excipients, disintegrators, buffering agents, emulsifying agents, suspending agents, soothing agents, stabilizers, preservatives, antiseptic agents, physiological saline and the like), or cells or components other than Muse cells that are present among MSCs, may be added to the cell preparation or pharmaceutical composition. A person skilled in the art may add such factors and chemical agents to the cell preparation and pharmaceutical composition in appropriate concentrations.

The number of Muse cells in the cell preparation and pharmaceutical composition to be prepared may be appropriately adjusted so as to obtain the desired effect for amelioration and/or treatment of perinatal brain damage (for example, improvement in learning disability or improvement in motor disability), in consideration of the target gender, age and body weight, the state of the affected area, and the state of the cells to be used. In Example 3 below, a perinatal hypoxic-ischemic encephalopathy (HIE) rat model was used to examine the effects of transplantation of Muse cells, and in a rat model of approximately 15 to 20 g body weight, a very excellent effect was obtained by administration of SSEA3 positive cells at 1 × 10⁴ cells/rat (individual). Based on these results, it is expected that for human neonates (within 28 days after birth), infants (less than 1 year after birth) or children (1 to 6 years after birth), administration of cells in an amount of approximately 3 × 10⁴ cells/kg to approximately 3 × 10⁷ cells/kg per individual, based on weight, should yield an excellent effect. For example, for an infant with a body weight of about 3,000 g, an estimated dose of about 1 × 10⁵ cells/individual to about 1 × 10⁸ cells/individual would be expected to be effective. However, in order to avoid an embolization by administration of cells into blood vessels, the SSEA-3 positive cells may be added to the cell preparation at no greater than 1 × 10⁷ cells/individual, for example, as the amount per single administration. Here, "individual" includes, but is not limited to, a rat or human. The cell preparation and pharmaceutical composition of the present invention may be administered several times (for example, 2 to 10 times) at appropriate intervals (for example, twice a day, once a day, twice a week, once a week or once every 2 weeks), until the desired therapeutic effect is obtained. Therefore, the therapeutically effective amount, while depending on the condition of the subject, is preferably a dose of 1 × 10⁴ cells to 1 × 10⁷ cells per individual, administered 1 to 10 times, for example. The total amount of administration per individual is not restricted, and may be 1 × 10⁵ cells to 1 × 10⁸ cells, 1×10⁵ cells to 5 × 10⁷ cells, 1 × 10⁵ cells to 1 × 10⁷ cells, 1 × 10⁵ cells to 5 × 10⁶ cells, 1 × 10⁵ cells to 1 × 10⁶ cells, 5 × 10⁵ cells to 1 × 10⁸ cells, 5 × 10⁵ cells to 5 × 10⁷ cells, 5 × 10⁵ cells to 1 × 10⁷ cells, 5 × 10⁵ cells to 5 × 10⁶ cells, 5 × 10⁵ cells to 1 × 10⁶ cells, 1 × 10⁶ cells to 1 × 10⁸ cells, 1 × 10⁶ cells to 5 × 10⁷ cells, 1 × 10⁶ cells to 1 × 10⁷ cells or 1 × 10⁶ cells to 5 × 10⁶ cells.

The target of amelioration and treatment by the cell preparation and pharmaceutical composition of the present invention is perinatal brain damage associated with learning disability and motor disability, and the period for administration may be after diagnosis of perinatal brain damage by neurological symptoms at birth, or ultrasonography or MRI of the head, and within several months immediately after diagnosis. According to the present invention, however, although the cell preparation is preferably administered immediately after injury, the effect of the cell preparation of the present invention may still be expected at a later period after injury, such as 1 hour, 1 days, 1 week, one month, 3 months or 6 months after injury, for example. Furthermore, since the Muse cells to be used have been confirmed in experiments by the present inventors to not elicit an immune response even when allogenically derived, they may be suitably administered until the desired effect for amelioration and treatment of perinatal brain damage is obtained. As demonstrated in Example 3 below, for improvement of perinatal brain damage by Muse cells using an HIE rat model, the therapeutic effect of improvement in perinatal brain damage over a long period (5 to 6 months after birth) tends to be more notable than the therapeutic effect over a short period (one month after birth), based on behavioral evaluation.

### 3. Creation of perinatal hypoxic-ischemic encephalopathy (HIE) rat model

An HIE rat model may be constructed and used to examine the amelioration and treatment effect on perinatal brain damage (for example, learning disability and motor disability) by the cell preparation of the present invention, as described herein. The rats to be used in the model are not restricted, and may generally be Wistar/ST rats or Sprague Dawley (SD) rats. Methods of creating HIE rat models are publicly known, and the HIE rat model may be created by the method of Rice et al. (Ann. Neurol., vol.9, 131-141(1981), incorporated herein by reference), for example. The site of infarct in a rat model created by this method can be evaluated using a two-dimensional imaging laser flowmeter or MRI to determine the presence of hypoxic ischemic encephalopathy.

The Muse cells to be used in the cell preparation and pharmaceutical composition of the present invention have the property of accumulating at disease sites. For administration of the cell preparation or pharmaceutical composition, therefore, there is no restriction on the mode of administration (for example, intraperitoneal, intramuscular, or local injection at the affected area), or the type of blood vessel for administration (vein or artery). The method for confirming whether the administered Muse cells have reached and engrafted to the affected site may be, for example, creation of Muse cells previously subjected to gene transfer so as to express a fluorescent protein (for example, green fluorescent protein (GFP)), and after administration to the body, observation may be carried out using a system that can detect fluorescence (for example, an IVIS^{R} Imaging System (product of Pharma International, Inc.)), to confirm of the dynamics of the Muse cells. Since the Muse cells to be used in the cell preparation and pharmaceutical composition of the present invention are human-derived, they are heterogenous with respect to rats. In an experiment in which allogenic cells are administered to an animal model, an immunosuppressive agent (such as cyclosporin) may be administered either before or simultaneously with administration of the allogenic cells to suppress *in vivo* rejection of the heterogenous cells.

### 4. Amelioration and treatment effect by Muse cells in HIE rat model

According to an embodiment of the present invention, the cell preparation and pharmaceutical composition of the present invention can ameliorate and/or treat perinatal brain damage in mammals including humans, and its various associated symptoms. Using an HIE rat model created as described above according to the present invention, improvement in symptoms by Muse cells in experimental perinatal brain damaged rats can be examined and the effect of the Muse cells can be evaluated. Specifically, the evaluation method may employ a common experimental system for evaluation of brain function using rats, examples of which include, for behavioral evaluation, the Rota-rod Test, Open Field Test, Shuttle Avoidance Test, Novel Object Recognition Test, Cylinder Test, Cat Walk Test and Morris Water Maze Test.

The "Rota-rod test" is a test using an apparatus that measures the coordination of motor function and static sense of a test animal. Specifically, the test animal is placed on an apparatus having a rotating rod that is capable of constant acceleration, and it is gradually accelerated from slow rotation. The time during which the test animal can walk to match the rotation without falling off from the rotating rod is determined. Repeated testing allows the motor learning function to be examined.

The "open field test" is based on placement of the test animal in a novel fixed space (for example, a 60 (W) × 60 (D) × 40 (H) cm box) and observation of the emotional behavior of the test animal, such as exploratory behavior. The observer may actually record the behavior of the test animal while photographing it with a video camera, extracting data and evaluating the indicators of activity/affectivity (for example, traveling distance, rest time and center dwelling rate).

The "shuttle avoidance test" is a test using an apparatus for observation of conditioned response behavior of a test animal to sound or light, and it allows evaluation of learning disability. With the test animal in one room, a sound is produced or a light is flashed, and then a current is circulated through the floor and the test animal escapes to another room to avoid the electrical stimulation. Repeating this procedure allows evaluation of the presence or absence of learning disability in the test animal.

In the "novel object recognition test", the test animal is allowed to freely explore a space in which two objects have been placed, and then one of the objects is replaced with a new object and the change in exploration time by the test animal for the new object is measured, to allow evaluation of memory learning and visual cognitive memory.

In the "cylinder test", the test animal is placed in a cylinder and the left/right difference in exploratory spontaneous motion of the paws on the wall of the cylinder is observed, to allow observation of motor function impairment.

In the "cat walk test", the test animal is caused to walk on a transparent glass plate with interior LED lighting, and video images of the foot soles taken from below with only the contact surface illuminated, based on the principle of total internal reflection, are analyzed by personal computer software, to allow evaluation of leg movement and the walking condition of the test animal.

The present invention will now be explained in more specific detail through the following examples, with the understanding that the present invention is in no way limited by the examples.

### EXAMPLES

### Example 1: Creation of perinatal hypoxic-ischemic encephalopathy (HIE) rat model and administration of Muse cells

The protocol for the experimental animals used in this research were approved by the Committee for Animal Experiments of Nagoya University, Faculty of Medicine. Wistar/ST rats (young) were obtained from Japan SLC, Inc. (Shizuoka Prefecture, Japan), and were placed in cages with free access to food and water and reared with a 12-hour light/dark cycle. The animal room and experimental space were kept at a constant 23°C.

An HIE rat model was created by the method described by Rice et al. (Ann. Neurol., vol.9, 131-141(1981)), using Wistar/ST rats (postnatal age of 7 days). Each rat was anesthetized by isoflurane inhalation. The left carotid artery was then doubly ligated and the ligated area was cut out. After resting for 1 hour with the mother, they were placed in a low oxygen environment of 8% O₂ at 37°C for 60 minutes, and then returned to their mother in the animal room that had been kept at 23°C. After 72 hours, the treatment group was administered Muse cells (1 × 10⁴ cells/individual) through the right carotid artery. In the control group, the left carotid artery was ligated in the same manner as the treatment group and a low oxygen state was provided, but instead of Muse cells, physiological saline alone was administered at the same volume. For the sham group, left carotid artery ligation and low oxygen were not carried out.

### Example 2: Preparation of Muse cells and confirmation of engraftment onto brain tissue

Muse cells were obtained by the method described in International Patent Publication No. WO2011/007900, relating to separation and identification of human Muse cells. In order to confirm engrafting to brain tissue of the Muse cells used for transplantation, the lentivirus-GFP gene was transferred into the Muse cells prior to administration so that the cells were labeled by green fluorescent protein (GFP). Cell populations containing the GFP-labeled Muse cells and Muse cells were separated by FACS, as GFP/SSEA-3 double positive cells. The Muse cells were then administered in the manner described in Example 1.

Engrafting to brain tissue by the Muse cells was confirmed in the brains of the 20-day-old rats that had been administered the Muse cells. After euthanizing the rats, the brains were excised and brain tissue slices were prepared by a common method. Primary antibody was reacted with GFP, and then peroxidase-labeled secondary antibody was reacted. Substrate for peroxidase (3, 3'-diaminobenzidine tetrahydrochloride) was added, and the Muse cells that had engrafted to the brain tissue were detected based on coloration (Fig. 1). The colored images of Fig. 1 show different regions (1 and 2) of sliced brain tissue (damaged left brain). The cells indicated by arrows at top are transplanted Muse cells. At bottom is shown a negative control that had not been reacted with antibody. Based on these results, Muse cells transplanted through the carotid artery of rats were confirmed to have engrafted onto damaged brain tissue. Similarly, rats were euthanized 2 weeks after administration of Muse cells and non-Muse cells in the same manner, and the engraftment of Muse cells in the affected hemisphere was confirmed (Fig. 12). It is seen that the Muse cells engrafted more notably to damaged brain tissue than in the system administered other.

### Example 3: Evaluation of improvement in perinatal brain damage by transplantation of Muse cells

Behavioral evaluation of an HIE rat model in which Muse cells had been transplanted was carried out in the following manner.

### (1) Rota-rod test

A commonly known apparatus (Rat Rota-Rod 47700 by UGO Basile) was used for measurement of the motor function coordination and static sense of test animals, to examine the improvement in perinatal brain damage by transplantation of the Muse cells. The test evaluation measures the time until the rats fall from a rotating stage, with repetition of the test (total of 4 times), and with the indicator of improvement in motor learning function being lengthening of the time until falling. The tested HIE rats were in 3 groups: a Muse cell-administered group, a group administered physiological saline alone and a sham group, and the evaluation was conducted for each over both a short period (one month after birth) and a long period (5 to 6 months after birth). The results are shown in Fig. 2. In both the short period and long period, the Muse cell-administered group showed improvement in motor learning disability function compared to the group administered physiological saline alone (vehicle) (Fig. 2, left), with a more notable improving effect being seen in the long period than in the short period (Fig. 2, right). These results suggest that Muse cells can improve motor learning disability function, as a type of perinatal brain damage.

Another Rota-rod test was conducted in the same manner as mentioned above for HIE rats (5 months after birth), with a Muse cell-administered group, a vehicle group and a sham group, and also a group administered non-Muse cells. The results are shown in Fig. 7. Improvement in motor learning disability function was seen in the Muse cell-administered group, but in the newly added non-Muse cell-administered group, no improvement in motor learning disability function was seen, similar to the vehicle. This suggested that Muse cells exhibit an excellent effect of improvement in motor learning disability function compared to non-Muse cells.

### (2) Open field test

Different types of rats were placed in a box having a novel fixed space, and the emotional behavior (hyperactivity, etc.) of the test animals was observed. The behavior of the test animals was photographed with a video camera (ANY-maze Video Tracking Software, product of Muromachi Kikai Co., Ltd.), and the traveling distance, immobile time and center section residence time of the test animals during a fixed period of time were measured (top, middle and bottom of Fig. 3, respectively). Rats placed in a novel space have longer traveling distance due to anxiety, but the traveling distance is reduced when they adapt to the space. When each rat group was compared, no significant difference was found between the vehicle and Muse group during the short period, but during the long period, reduced activity was seen in the Muse group compared to the vehicle group, with a similar tendency also found in the sham group as well. This suggested that administration of Muse cells can improve emotional behavior of test animals.

Another open field test was conducted in the same manner as mentioned above for HIE rats (5 months after birth), with a Muse cell-administered group, a vehicle group and a sham group, and also a group administered non-Muse cells. The results are shown in Fig. 8, with the mean speed of the test animals also being measured. In regard to the mean speed, the speed of movement of rats placed in a novel space increases in proportion to anxiety, while the speed is lower with low or lack of anxiety. Notable improvement was seen in the Muse cell-administered group in all of the test systems, but in the newly added non-Muse cell-administered group, no improvement in emotional behavior was seen, similar to the vehicle. This suggested that Muse cells exhibit an excellent effect of improvement in emotional behavior compared to non-Muse cells.

### (3) Shuttle avoidance test

A shuttle avoidance test was conducted to evaluate improvement in learning disability with different types of rats. The apparatus used for the evaluation was a MED-PC Version IV/MED-APA-DIM by Med Associates Inc., and the avoidance rate of the rats to electrical stimulation was measured a total of 5 times (Fig. 4), by a common method. The rats in all of the groups learned to avoid the stimulation, but a significant difference was seen in the Muse cell group compared to the vehicle group in both the short period and the long period. The avoidance rate of the Muse cell group was also about the same as that of the sham group. This suggested that administration of Muse cells improves learning disability of test animals.

Another shuttle avoidance test was conducted in the same manner as described above for HIE rats (5 months after birth), with a Muse cell-administered group, a vehicle group and a sham group, and also a group administered non-Muse cells. The results are shown in Fig. 9. Improvement in learning disability was seen in the Muse cell-administered group, but in the newly added non-Muse cell-administered group, no improvement in learning disability was seen, similar to the vehicle. This suggested that Muse cells exhibit an excellent effect of improvement in learning disability compared to non-Muse cells.

### (4) Novel object recognition test

A commonly employed apparatus was used for the novel object recognition test (ANY-maze Video Tracking Software, product of Muromachi Kikai Co., Ltd.). Different rats were allowed to freely explore a space in which two objects (familiar objects) had been placed, for a fixed period of time, after which one of the objects was replaced with a novel object, and the difference in exploration time of the rat for the new object was measured. The DI (Discrimination Index) was used as the index for evaluation. Specifically, calculation was by DI = (TN - TF)/(TN + TN) (where TF: exploration time for familiar object, TN: exploration time for novel object). A higher DI value indicates higher interest for the object. In the short period, the Muse cell group had a significant difference over the vehicle group, but the DI was still <0, indicating less interest in the novel object. In the long period, however, the Muse cell group had a significant difference over the vehicle group and had increased interest for the novel object similar to the sham group (Fig. 5). These results suggested the possibility that memory learning or visual cognitive memory is improved by administration of Muse cells.

Another shuttle avoidance test was conducted in the same manner as described above for HIE rats (5 months after birth), with a Muse cell-administered group, a vehicle group and a sham group, and also a group administered non-Muse cells. The results are shown in Fig. 10. High interest for the novel object was exhibited in the Muse cell-administered group, but in the newly added group which had been administered the non-Muse cells, no such notable effect was seen as in the Muse cell-administered group. This suggested that Muse cells exhibit an effect of increasing interest in novel objects, as compared to non-Muse cells.

### (5) Cylinder test

Different rats were placed in a cylinder (diameter: 20 cm), and the right/left difference in exploratory activity of the paws on the wall between the healthy side (left paw) and affected side (right paw) was confirmed. The index used for the evaluation was "non-impaired paw preference". Specifically, calculation was: Non-impaired paw preference (%) = (R - L)/(R + L + B) × 100 (where R: use frequency of right paw, L: use frequency of left paw, B: frequency use of both paws). A higher value indicates higher frequency of use of the right paw (affected side) when upright. The results for the long period are shown in Fig. 6A. The results show a significant difference for the Muse cell group compared to the vehicle group, and very high frequency of use of the right paw (affected side). This suggested that motor function is improved by administration of Muse cells.

Fig. 6B is a more common representation of Fig. 6A as used in research articles. Specifically, calculation was: Non-impaired paw preference (%) = (L - R)/(R + L + B) × 100 (where R: use frequency of right paw, L: use frequency of left paw, B: frequency use of both paws). A higher value indicates higher frequency of use of the left paw (healthy side) when upright.

Another cylinder test was conducted in the same manner as described above for HIE rats (5 months after birth), with a Muse cell-administered group, a vehicle group and a sham group, and also a group administered non-Muse cells. In this test, the healthy side of the rats was the right paw and the affected side was the left paw. The results are shown in Fig. 11. Improvement in motor function was seen in the Muse cell-administered group, but in the newly added non-Muse cell-administered group, no improvement in motor function was seen, similar to the vehicle. This suggested that Muse cells exhibit an excellent effect of improvement in motor function compared to non-Muse cells.

### INDUSTRIAL APPLICABILITY

The cell preparation and pharmaceutical composition of the present invention can be applied for amelioration and treatment of perinatal brain damage such as learning disability and motor disability, by administration to an HIE rat model.

All of the publications and patent literature cited herein are incorporated into the present specification in their entirety as reference. The specific embodiments of the present invention were explained in the present specification for the purpose of example, and it will be easily appreciated by a person skilled in the art that various modifications may be employed such as are not outside of the spirit and scope of the present invention.

## Claims

1. A cell preparation for amelioration and/or treatment of perinatal brain damage, comprising pluripotent stem cells positive for SSEA-3 isolated from mesenchymal tissue of or cultured mesenchymal cells.

2. The cell preparation according to claim 1, comprising a cell fraction wherein pluripotent stem cells positive for SSEA-3 have been concentrated by external stress treatment.

3. The cell preparation according to claim 1 or 2, wherein the pluripotent stem cells are CD105-positive.

4. The cell preparation according to any one of claims 1 to 3, wherein the pluripotent stem cells are CD117-negative and CD146-negative.

5. The cell preparation according to any one of claims 1 to 4, wherein the pluripotent stem cells are CD117-negative, CD146-negative, NG2-negative, CD34-negative, vWF-negative and CD271-negative.

6. The cell preparation according to any one of claims 1 to 5 above, wherein the pluripotent stem cells are CD34-negative, CD117-negative, CD146-negative, CD271-negative, NG2-negative, vWF-negative, Sox10-negative, Snail-negative, Slug-negative, Tyrp1-negative and Dct-negative.

7. The cell preparation according to any one of claims 1 to 6, wherein the pluripotent stem cells have all of the following properties:
(i) low or non-existent telomerase activity;
(ii) having the ability to differentiate into any of the three germ layers;
(iii) exhibiting no neoplastic proliferation; and
(iv) having self-renewal ability.

8. The cell preparation according to any one of claims 1 to 7, wherein the perinatal brain damage is selected from the group consisting of learning disability, motor disability, cerebral palsy, behavior disorder, mental development abnormality, sensory disorder, speech disorder, epilepsy, dysphagia and abnormal respiratory control.

9. The cell preparation according to any one of claims 1 to 8, wherein the pluripotent stem cells have the ability to engraft into brain tissue.

10. The cell preparation according to any one of claims 1 to 9, which is to be administered to a human neonate, infant or child with the pluripotent stem cells at from approximately 1 × 10⁵ cells/individual to approximately 1 × 10⁸ cells/individual, as the therapeutically effective amount.

11. The cell preparation according to any one of claims 1 to 10, which is to be administered to a human neonate, infant or child with the pluripotent stem cells in an amount of cells per body weight of approximately 3 × 10⁴ cells/kg to approximately 3 × 10⁷ cells/kg per target individual, as the therapeutically effective amount.
